# EUROPEAN PATENT SPECIFICATION

(11) **EP 1 740 092 B1**
(45) Date of publication and mention of the grant of the patent: **10.06.2015**
(21) Application number: 05744774.0
(22) Date of filing: 20.04.2005
(51) Int. Cl.: A61B 5/00

(54) **PASSIVE SENSOR WITH WIRELESS TRANSMISSION**
PASSIVSENSOR MIT DRAHTLOSER ÜBERTRAGUNG
DETECTEUR PASSIF A TRANSMISSION SANS FIL

(30) Priority: 27.04.2004 US 565510 P
(43) Date of publication of application: 10.01.2007
(73) Proprietor: ETH Zurich, 8092 Zürich (CH)
(72) Inventor: HIEROLD, Christofer, CH-5400 Baden (CH); WENDLANDT, Michael, Eric, CH-8004 Zürich (CH); UMBRECHT, Florian, CH-8005 Zürich (CH)
(74) Representative: Müller Hoffmann & Partner
(86) International application number: PCT/EP2005/004204
(87) International publication number: WO 2005/102151

(56) References cited:
- EP-A- 0 751 384
- EP-A- 0 751 384
- EP-A- 1 356 765
- EP-A- 1 356 765
- DE-A1- 2 310 737
- DE-A1- 2 310 737
- DE-A1- 3 405 086
- DE-A1- 3 405 086
- DE-C- 207 940
- FR-A- 540 719
- US-A1- 2003 100 822

## Description

The invention relates to a passive sensor according to the preamble of claim 1.

Most in situ/in vivo sensors for monitoring parameters within the living body require wires in order to facilitate read out and communication with the sensor. While effective, such sensor arrangements, and especially the wires penetrating the skin, can cause severe patient discomfort, inflammation, and infection. To address these negative effects, wireless sensors have been introduced.

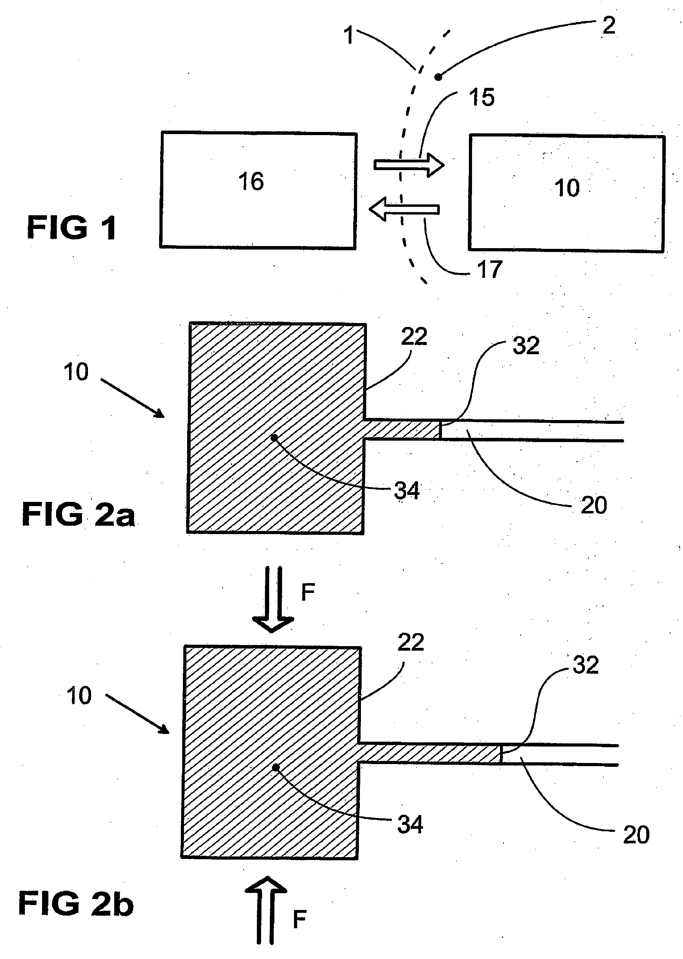

The principle of a wireless sensor system is shown in figure 1. Such sensors 10, e.g. strain sensors, are placed in a living body 2. The skin is symbolically shown with a line and denoted by the reference numeral 1. The strain sensor 10 is interrogated via a request signal 15 sent out from a transmitter/receiver/evaluation unit 16. The unit 16 receives a response signal 17 in order to derive the actual size of the strain.

In wireless sensor systems the sensor unit itself can be designed active, semi-passive or passive:
- Whereas «active» means that the sensor unit consists of active circuits powered by an on board power supply (e.g. battery).
- «Semi-passive» sensor units consist of active circuits which are powered by inductive coupling or an RF signal from the request unit.
- «Passive» devices don't have active circuits and employ a distortion of the received request signal as response signal.

Sensor-systems, e.g. for mechanical axial strains, are reported in various publications for medical [1, 2] and non-medical applications [2-5].

So far only inductor/capacitor circuit (LC) resonators have been described as passive sensor devices in medical applications performing over a range of several millimeters [1, 2].

### Brief discussion of available wireless sensor.systems

### 1. LC resonator sensor [1,2]

The sensor principle is a LC resonator sensor. Compressive strain changes the actual cross-section of the solenoid or the gap distance of the capacitor, thus changing its inductance or capacity respectively.

### 2. Surface acoustic wave sensor SAW [3,4]

A passive acoustic sensor principle, like a surface acoustic wave device SAW, re-transmits a linearly distorted version of the radio request signal: a RF interrogation signal is re-ceived by the interdigital transducer, e.g. a piezoelectric material, and transformed into a surface wave, which is affected by changes in the surface properties. The surface wave is reflected and then reconverted into an electrical signal and transmitted by the antenna.

### 3. Magneto-elastic sensor [5]

The magneto-elastic sensor mechanically deforms when subjected to a magnetic field impulse, launching elastic waves within the sensor the magnitude of which is greatest at the mechanical resonant frequency. Through the inverse magneto-elastic effect, the vibration of the sensor generates a time varying magnetic flux, which can be measured wireless with a set of pick-up coils. In addition, the mechanical vibrations also generate an acoustic wave that can be detected with a microphone. The inverse relation between the length, modulus, or mass of the sensor vibrating in its basal plane and the longitudinal resonance frequency allows the determination of various parameters on a sub-micro scale, e.g. strain, mass loads, liquid viscosity, or temperature. Magneto-elastic sensors are typically made of amorphous ferromagnetic metallic glasses such as Fe₄0Ni₃₈Mo₄B₁₈ or Fe₈B_{13.5}Si_{3.5}C₂, which generally miss biocompatibility.

### Materials for sensors in medical applications

When used for medical applications, like in situ/in vivo monitoring of strains occurring along a bone of the human skeleton, a strain sensor should fulfil several requirements to minimize the impact on the patient. These requirements are first of all biocompatibility or biocompatibility and biodegradability, which are related to the micro-design of the sensor and the choice of materials. Most of the materials used for the state-of-the art techniques, however, are not biocompatible or biodegradable and a potential system would have to be encapsulated.

Recently a new class of highly biocompatible or biocompatibility and biodegradability polymeric materials has emerged [7-9]. These materials already had great impact on medical applications like artificial hip joints, biodegradable implants, which provide secure initial fixation strength while allowing degradation and replacement by the host tissue, or biodegradable drug delivery systems.

### Sensor design / micro-structuring

To provide for maximum biocompatibility when used as medical implant, micro-design of the sensor is preferred, which includes material characterization and micro-structuring of the materials. Usually microfabrication uses photolithographic technologies , which are restricted to photosensitive materials only. Biocompatible/-degradable polymers are usually not photosensitive, hence, non-photolithographic technologies like microprinting, replica molding, microembossing, or microcutting are preferred and have been demonstrated for thermoplastic polymers as fabrication method for generating sub-micrometer features [10-15]. Thermoplastic polymers include the class of amorphous and semi-cristalline polymeric solids, which soften when heated above the glass transition temperature (amorphous polymers) or melting temperature (semi-cristalline polymers).

Wireless sensors however, were not initially designed for implantation within the human body as the required components are or may be electronic and toxic.

The present invention therefore addresses the problem of creating a wireless passive sensor, which uses a transmission principle beyond RF-signals and which doesn't need any electrical circuitry. The latter provides the option to built the sensor entirely from biodegradable materials. Furthermore it is an object of the present invention to create a sensor, which detects a certain parameter, e.g. a change in length or temperature, with a high accuracy as well as high resolution.

These objectives are solved by a sensor according to the features specified in claim 1.

Advantageous configurations and applications of the invention are specified in further claims.

Objectives and advantages of the present invention as well as the preferred embodiments include the following:
- to provide a sensor which may be used within the body and communicated with from outside the body;
- to create a signal amplification system which may be used with a variety of signal generating means, within the body, the amplification system being detectable from outside the body;
- to create an individual sensor and/or amplification system made of bio-compatible and/or bio-degradable materials so as to facilitate a reduction in the number of required surgeries;
- to set out a method for using the above mentioned invention; and
- to implement the aforementioned using low cost materials so as to facilitate mass production and disposability.

A preferred task of the wireless in situ passive strain sensor (acronym: WIPSS) is to detect in situ/in vivo mechanical strains via a low-cost, implantable, biocompatible and biodegradable, micro-designed, wireless and passive sensor unit.

The concept of the sensor unit is to transmit small relative strains, typically occurring in the human body of the order 0.00001 to 0.001 according to the indications in [6], via an amplifycation system into a signal which can be detected through human tissue by available ultrasound or other imaging technologies via radiation.

The strain sensor according to the present invention requires no power supply and may be composed of biocompatible or bio-compatible and biodegradable materials thereby obviating the need for surgical removal. The sensor unit may have a micro design so that it may be implemented in a variety of uses. One use is on a bone wherein the sensor is directly connected to the bone via known surgical techniques such as gluing. The micro-size facilitates localized positioning. The use of biocompatible or biocompatible and biodegradable materials causes low costs and facilitates disposability and mass production.

Biocompatible and biodegradable materials are known in the art and may be used as a base material for the present invention. Such materials include DEGRAPOL, polyglcolide, poly-lactide, poly(*ε*-caprolactone), pol(lactide co-glycolide), and other materials as set out in the article Middleton et al.. Biocompatible materials include: Polyvinylchloride, Polyethylene, Polymethylmethacrylate, Polyamides and the like. Reference is made to [16] an [17]. In addition, the materials used for the components of the present invention may comprise all materials, such as polymers, but not limited to, biocompatible or biocompatible and biodegradable materials, that are capable of transfering stress into strain and therefore into a change in volume of the sensor's body.

The amplification unit is readable from outside the body by conventional ultrasound techniques. Other reading techniques may be used as known to one skilled in the art as radiation e.g. in the visible or near visible range. Ultrasound, as is known, reads through human tissue and can «see» liquid gas and solid gas interfaces. The liquids may also be biodegradable and biocompatible as per the discussion above. Liquid is also included in the sensing unit such that deformation of the sensing unit urges the liquid along a track in the amplification unit, the track being imageable from outside the body. Alternatively, micro-gear systems may be used with a gas bubble at a lever's end for an indicator. The lever may pivot in response to deformation, the mechanical force of which is directly transmitted to the lever. The mechanical force may originate from a sensor unit wall. Accordingly, more than one lever responding to more than one wall may be used. Additionally more than one bubble may be employed.

The imaging may be a pixel maze of 100 pixels. The amplification unit may include micro-channels, also called micro-tubes, for the liquid to flow in. The actual layout and design of the micro-channels is a design choice dependent upon application.

The invention will become apparent upon consideration of the following detailed description of specific embodiments thereof with a reference to a drawing in which:
- Figure 1: principle of a wireless sensor system according to the state of the art;
- Figure 2a, 2b, 2c: principle of the signal amplification according to two embodiments of the present invention;
- Figure 3: another embodiment of a mechanical strain amplification;
- Figure 4: an embodiment of a hydro-mechanical strain amplification;
- Figure 5a, 5b: a further embodiment of a hydro-mechanical strain amplification sensing or compensation effects of temperature.

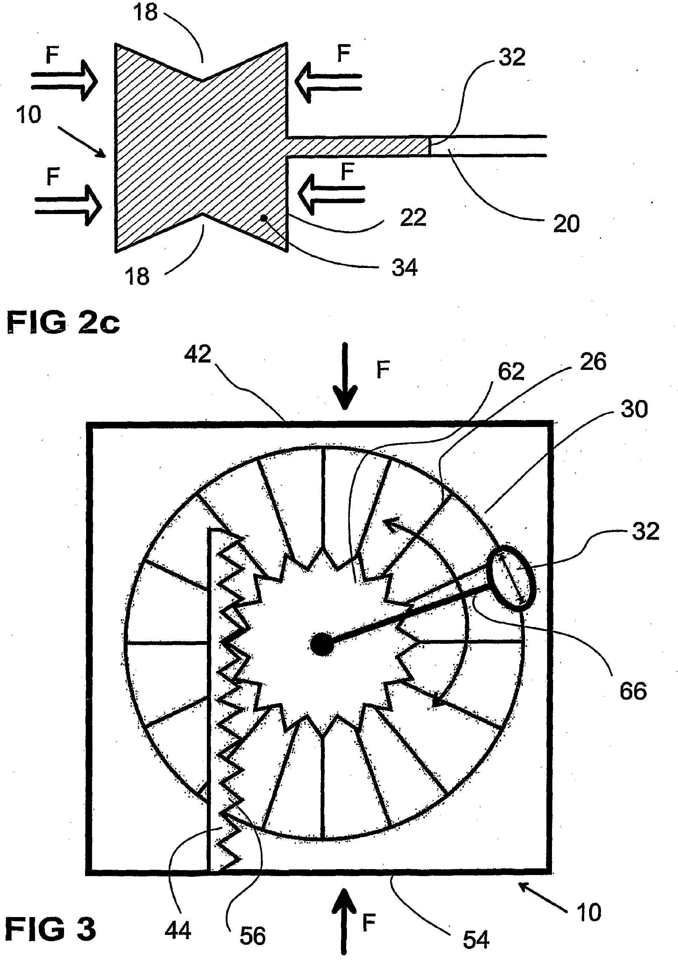

The principle of the strain amplification according to the following depicted embodiment is show in figure 2a and figure 2b for a sensing unit 10. A reservoir 22 contains an incompressible liquid 34. The reservoir 22 has an opening to a micro tube 20. Under a force F, see fig. 2b, the volume of the reservoir will be decreased and there will be a corresponding change of the position of an liquid/gas interface. Figure 2c shows a similar embodiment of a sensing unit 10 with a reservoir 22. Recesses 18 enhance the decrease of the volume, when a force F is applied to the side walls, this kind of recesses are also called accordion principle.

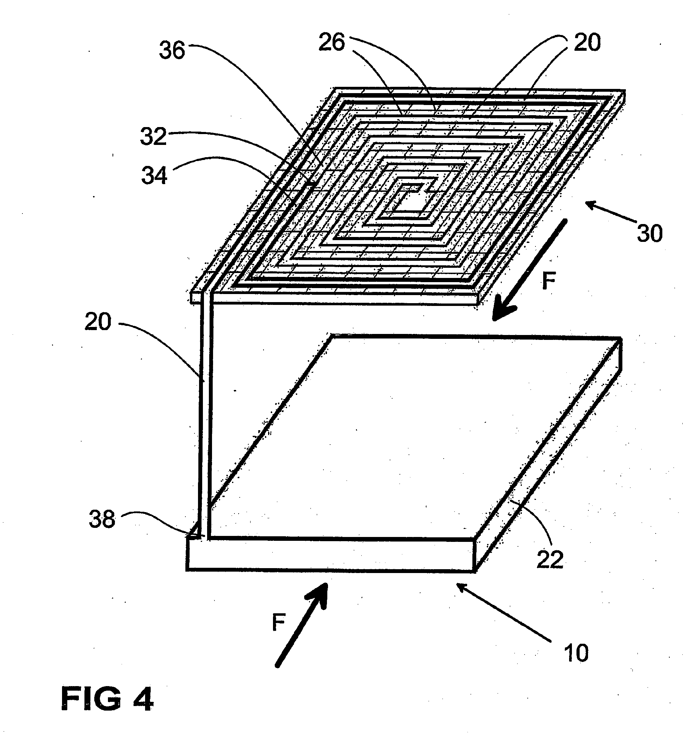

An concrete embodiment of the invention is shown in figure 4. A sensing unit 10 comprises a liquid containing reservoir 22. The reservoir 22 may comprise biocompatible and/or biodegradable polymers such discussed above. Other polymers may be used or substituted provided that they are bio-compatible and/or biodegradable.

The size and shape of the reservoir 22 is dependent upon application with other designs than the one depicted available to one skilled in the art. As depicted, the reservoirs 22 compress a substantial square-like structure having an upper and lower surface with a side length of about 5mm. The reservoir 22 is three dimensional with a width of about 2 mm. The reservoir materials must be biocompatible, with the following mechanical properties. The reservoir mechanical properties should not be to stiff and be easily coupled to the bone. The sensing unit may be transparent or opaque.

A micro-channel 20 connects the reservoir 22 with a display unit 30. The channel 20 may have a variable length depending upon the location of the display unit 30. Accordingly, the display unit could be placed directly on the sensing unit 30, or remote from the reservoir 22. The latter could be preferable for applications several centimeters inside the body, where it might be preferable for the display unit 30 to be placed substantially below the skin. Likewise, depending upon application, the channel 20 may be several millimeters, centimeters and so forth without limit but for the physical properties of the components. The material for the channel 30 may be the same as the reservoir 22 or at least within the same family of materials.

The display unit 30 may comprise the same material or at least same family of materials as the sensing unit 10. The display unit 30 comprises a housing having a micro-channel path 20 running therein. The path design is a matter of design choice provided that the resolution of the outer body sensing device is able to resolve locations along the path. As shown in figure 4, a spiral layout of the channel 20 is provided. Other layouts as e.g. a meandric layout are also applicable. State of the art microstructing fabrication techniques such as photo and non-photolithography or soft photolithography may be used to fabricate the path. The micro-channel 20 is fabricated so as to accommodate liquid 34 from the reservoir 22 therein and therethrough. As shown in the figure, gradations 26 are included on the surface of the display unit 30. While such may be part of the fabrication process, as depicted, such are the result of imaging techniques to be discussed below. The reservoir 22 is liquid tight during sensor operation and include liquid therein. The actual amount of liquid as well as the liquid itself is a matter of design choice, provided the composition and amount do not rise to the level of toxicity. Preferably, the liquid is biocompatible or biocompatible and biodegradable.

In operation, e.g. as strain sensor, the sensing unit 10 is positioned at a location where the force F is of interest. One location of operation is within the human body, including attachment directly to or within a bone. Because of the presence of the micro-channel 20, the display unit 30 may be remotely located from the sensing unit 10. Accordingly, the sensing unit 10 is placed and arranged such that forces F cause at least one wall of the sensing unit to deform. The deformation in turn forces a comparably equal amount of liquid from the reservoir 22. The liquid is received via micro-channel 20. Accordingly, the liquid forced from reservoir 22 by the wall deformation directly increases the amount of liquid located in the micro-channel 20 and display channel 20 thereby pushing the forward liquid/gas interface 32 further along path. As the volume or amount of liquid 34 in the display channel 20 increases, more and more of the channel 20 becomes occupied such that the location of the end point 32 of the liquid within the display channel 20 is an indication of the amount of deformation occurring at the reservoir 22 and accordingly, the amount of stress F present in the area surrounding the reservoir 22.

Imaging means, such as ultrasound waves reflected by the liquid/gas interface 32 may be used from outside the body to detect the location of the liquid end point. Imaging techniques may be used to apply a grid like image over the path such that units of measurements and readable displays may result.

In a second embodiment, as shown in figure 3, the display unit may not be remotely located from the sensing unit 10. Herein, direct mechanical coupling between sensing unit 10 and display unit 30 are used with the walls of the sensing unit 10 also defining the outer boundaries of the display unit 30. Other relations between sensing and display unit walls may be employed without departing from the spirit of the invention. The sensing unit 10 is depicted as a square having substantially equal-length sides. Other geometrical shapes may be employed, as would be envisioned by one skilled in the art, as may be required for example upon application. For example, with some applications a trapezoidal or triangular shape may be substituted. Returning to figure 3, a micro rod 44 is mechanically connected to a side wall 54. The micro rod 44 is so arranged so as to displace with displacement of side wall 54. For purposes of clarify, only one micro rod affixed to a single wall is shown. An additional number of gear rods 44 attached to the same or other side walls may be used depending upon application. The additional gear rods 44 facilitate stress detection from other directions. The display unit 30 comprises a surface bordered by the sensing unit walls. Located on the display unit is a rotating gear 62. The rotating gear 62 is depicted as a full circle, although, semi-circles and other geometrical designs comprising one or more gears which convert the lateral displacement of the gear rod 44 into a circular displacement may be employed. Rotating gear 62 includes teeth 56, sized and spaced so as to mechanically cooperate with the micro gear teeth 56 such that lateral displacement of the micro gear 44 is mechanically converted into rotational displacement by the cooperation of the two gears teeth 56. Affixed to rotating gear 62 is a display arm 66. Display arm 66 is arranged to rotate with the rotating gear 62 and may further be affixed to a midpoint or pivot of the rotating gear 62. At the other end of the arm is a display. As depicted, the display is an encased gas bubble, that is a liquid/gas interface 32. Other displays may be used provided they are non-toxic, biocompatible or biocompatible and biodegradable and detectable by non-evasive means from outside the body. As in known in the art, ultra-sound detected the interface of liquid and gas 32 thereby making the displacement of display visible from outside the body. The side walls may have a length of 6 millimeters and the display a width of 0.4 millimeters. The sensing unit walls 54 are elastic and comprised of the same materials as those employed above with the first embodiment.

Known ultrasound imaging techniques may be employed to detect the location and displacement on a display 30. The mapable display includes radial gradations 26 which may be used to determine rotational displacement of the display. Should the sensor wall 54 deformation be converted into another mechanical displacement, appropriate mapping would be used to detect the respective display.

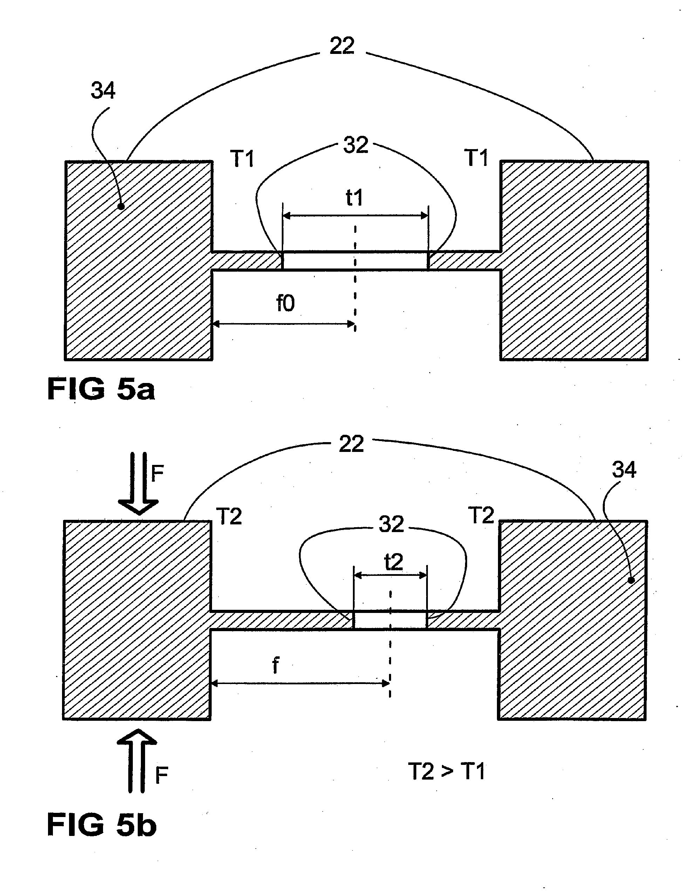

Figure 5a shows an embodiment based on the principle according to the figures 2a, 2b or 2c. Two reservoirs 22 each containing an incompressible liquid are coupled via a channel. Assumed is no strain nor force on neither reservoir 22, but this embodiment is exposed in an environment of a temperature T1. By changing the temperature T1 the distance t1 between the two liquid/air interfaces 32 changes accordingly, that is an increase of temperature leads to a decrease of the distance t1 and vice-versa. This principle serves as a base for a thermometer. The location of the said liquid/air interface 32 either - one or both - will be detected with radiation. The location of the center of the two liquid/air interfaces 32 remains unchanged during a change of temperature. The before mentioned location is represented by a distance f0.

According to figure 5b a strain F acts on the left reservoir 22 causing a decrease of its volume. The liquid/air interface moves to the right. The distance f of the middle of said liquid/air interface represents a measure for the strain F acting on the left reservoir 22. Furthermore the embodiment according to figure 5b is exposed in an environment of a temperature T2 > T1. Since the right hand reservoir 22 is also exposed to the same temperature T2, the influence of temperature is separated, only the distance t2 changes according to the change of temperature but not the distance f.

The present sensing unit 10 may be used with other display or amplification units and viceversa.

### List of reference numerals, glossary

- 1: Skin
- 2: part of living body
- 10: Sensing unit, sensor unit, strain sensor
- 12: Lower surface
- 14: Lower surface
- 15: Request signal, power transmission
- 16: Transmitter/receiver/evaluation unit
- 17: Response signal
- 18: recess, indentation
- 20: indication means; micro channel, micro tube, display channel, channel, tube
- 22: first means responsive to a physical impact; reservoir
- 26: gradation
- 28: surface
- 30: display unit, display
- 32: contrast interface; liquid/gas interface, liquid end point
- 34: incompressible liquid
- 36: gas, gas
- 38: outlet into micro-channel 20
- 40: spiral
- 42: elastic container
- 44: indication means; gear rod, micro gear
- 50: sensing unit
- 54: side wall
- 56: teeth
- 62: indication means, rotating gear
- 66: display arm, lever

### List of used acronyms and symbols

- F: force
- f, f0: length representing a force
- T1, T2: temperature
- t1, t2: length representing a temperature
- WIPSS: wireless in situ passive strain sensor

### References

[1] L. Rosengren, P. Rangsten, Y. Backlund, B. Hok, B. Svedbergh, and G. Selen, "A System for Passive Implantable Pressure Sensors," Sensors and Actuators a-Physical, vol. 43, pp. 55-58, 1994.
[2] J. C. Butler, A. J. Vigliotti, F. W. Verdi, and S. M. Walsh, "Wireless, passive, resonant-circuit, inductively coupled, inductive strain sensor," Sensors and Actuators a-Physical, vol. 102, pp. 61-66, 2002.
[3] W. E. Bulst, G. Fischerauer, and L. Reindl, "State of the art in wireless sensing with surface acoustic waves," Ieee Transactions on Industrial Electronics, vol. 48, pp. 265-271, 2001.
[4] A. Pohl, "A review of wireless SAW sensors," Ieee Transactions on Ultrasonics Ferroelectrics and Frequency Control, vol. 47, pp. 317-332, 2000.
[5] C. A. Grimes, C. S. Mungle, Z. F. Zeng, M. K. Jain, W. R. Dreschel, M. Paulose, and K. G. Ong, "Wireless magnetoelastic resonance sensors: A critical review," Sensors, vol. 2, pp. 294-313, 2002.
[6] D. B. Burr, C. Milgrom, D. Fyhrie, M. Forwood, M. Nyska, A. Finestone, S. Hoshaw, E. Saiag, and A. Simkin, "In vivo measurement of human tibial strains during vigorous activity," Bone, vol. 18, pp. 405-410, 1996.
[7] J. C. Middleton and A. J. Tipton, "Synthetic biodegradable polymers as orthopedic devices," Biomaterials, vol. 21, pp. 2335-2346, 2000.
[8] A. Lendlein, P. Neuenschwander, and U. W. Suter, "Tissue-compatible multiblock copolymers for medical applications, controllable in degradation rate and mechanical properties," Macromolecular Chemistry and Physics, vol. 199, pp. 2785-2796, 1998.
[9] B. Saad, Y. Kuboki, M. Welti, G. K. Uhlschmid, P. Neuenschwander, and U. W. Suter, "DegraPol-Foam: A degradable and highly porous polyesterurethane foam as a new substrate for bone formation," Artificial Organs, vol. 24, pp. 939-945, 2000.
[10] Y. N. Xia and G. M. Whitesides, "Soft lithography," Annual Review of Materials Science, vol. 28, pp. 153-184, 1998.
[11] N. Stutzmann, T. A. Tervoort, C. W. M. Bastiaansen, K. Feldman, and P. Smith, "Solid-state replication of relief structures in semicrystalline polymers," Advanced Materials, vol. 12, pp. 557-562, 2000.
[12] N. Stutzmann, T. A. Tervoort, D. J. Broer, H. Sirringhaus, R. H. Friend, and P. Smith, "Microcutting materials on polymer substrates," Advanced Functional Materials, vol. 12, pp. 105-109, 2002.
[13] G. Fichet, N. Stutzmann, B. V. O. Muir, and W. T. S. Huck, "Microembossing of elastomeric triblock copolymers," Advanced Materials, vol. 14, pp. 47-51, 2002.
[14] D. K. Armani and C. Liu, "Mircofabrication technology for polycaprolactone, a biodegradable polymer," Journal of Micromechanics and Microengineering, vol. 10, pp. 80-84, 2000.
[15] D. V. McAllister, P. M. Wang, S. P. Davis, J. H. Park, P. J. Canatella, M. G. Allen, and M. R. Prausnitz, "Microfabricated needles for transdermal delivery of macromolecules and nanoparticles: Fabrication methods and transport studies," Proceedings of the National Academy of Sciences of the United States of America, vol. 100, pp. 13755-13760, 2003.
[16] J. B. Park, J. D. Bronzino: Biomaterials-Principles and Applications. Pp. 55-77. 2003, CRC Press LLC.
[17] Shastri V.P.: Non-Degradable Biocompatible Polymers in Medicine: Past, Present and Future Current Pharmaceutical Biotechnology, October 2003, vol. 4, no. 5, pp. 331-337(7).

## Claims

1. A sensor (10), comprising
- first means (22, 42) responsive to a physical impact (F, T1, T2) thereupon; and
- indication means (20; 44, 62) coupled to the first means (22, 42), 'the indication means indicating the magnitude of the physical impact (F, T1, T2),
wherein
the sensor (10) comprises a reservoir (22) and the indication means (20; 44, 62) comprises a micro-channel (20) for receiving liquid (34) from the reservoir (22) urged from the reservoir (22) by the physical impact (F, T1, T2),
**characterized in that** the indication means (20; 44, 62) are built by a contrast interface (32) contrasting two mediums and which contrast interface (32) is readable by sound.

2. The sensor (10) according to claim 1,
wherein
one of the two mediums is a liquid (34) and the other one is a gas.

3. The sensor (10) according to anyone of the claims 1 or 2,
wherein
the micro-channel (20) comprises a spiral or a meandric layout.

4. The sensor (10) according to anyone of the predeeding claims,
wherein
the micro-channel (20) comprises a maximum width of 1 mm, a maximum height of 0.1 mm and a maximum length of 100 mm.

5. The sensor (10) according to anyone of the predeeding claims,
wherein
micro-channel (20) comprises a minimum width of 0.01 mm, a minimum height of 0.0001 mm and a minimum length of 10 mm.

6. The sensor (10) according to anyone of the claims 1 to 5,
wherein
a second reservoir (22) is connected with the channel (20) in order to separate indication means (32) indicating different physical impacts (F, T1, T2).

7. The sensor (10) according to claim 6,
wherein
the channel (20) contains two liquid/air interfaces (32) where the distance (t2) between the two liquid/air interfaces (32) represents the temperature (T1, T2) of the sensor (10).

8. The sensor (10) according to claim 6 or 7,
wherein
the channel (20) contains two liquid/air interfaces (32) where the distance (f) between one liquid/air interfaces (32) and one reservoir (22) represents the strain (F) applied to that reservoir (22).

9. The sensor (10) according to anyone of the claims 1 to 7,
wherein
the reservoir (22) has at least one recesse (18) in order to increase the volume change when a strain (F) is applied on that reservoir (22).

10. The sensor (10) according to anyone of the claim 1 to 9,
wherein
the sensor (10) is biocompatible.

11. The sensor (10) according to claim 10,
wherein
the (10) sensor is biodegradable.

## Patentansprüche

1. Sensor (10) mit
- ersten Einrichtungen (22, 42), die auf einen auf sie gerichteten physikalischen Einfluss (F, T1, T2) reagieren; und
- Anzeigeeinrichtungen (20; 44, 62), die mit den ersten Einrichtungen (22, 42) gekoppelt sind, wobei die Anzeigeeinrichtungen die Größe des physikalischen Einflusses (F, T1, T2) anzeigen, wobei
der Sensor (10) ein Reservoir (22) aufweist, und die Anzeigeeinrichtungen (20; 44, 62) einen Mikrokanal (20) zum Empfangen von Flüssigkeit (34) aus dem Reservoir (22) aufweisen, die durch den physikalischen Einfluss (F, T1, T2) aus dem Reservoir (22) gedrängt wird,
**dadurch gekennzeichnet, dass**
die Anzeigeeinrichtungen (20; 44, 62) durch eine Kontrastgrenzfläche (32) ausgebildet sind, die zwei Medien kontrastiert, wobei die Kontrastgrenzfläche (32) durch Schall lesbar ist.

2. Sensor (10) gemäß Anspruch 1, wobei
eines der beiden Medien eine Flüssigkeit (34) und das andere ein Gas ist.

3. Sensor (10) gemäß einem der Ansprüche 1 oder 2, wobei
der Mikrokanal (20) eine spiralförmige oder mäandernde Gestaltung aufweist.

4. Sensor (10) gemäß einem der vorherigen Ansprüche, wobei
der Mikrokanal (20) eine maximale Breite von 1 mm, eine maximale Höhe von 0,1 mm und eine maximale Länge von 100 mm aufweist.

5. Sensor (10) gemäß einem der vorherigen Ansprüche, wobei
der Mikrokanal (20) eine minimale Breite von 0,01 mm, eine minimale Höhe von 0,0001 mm und eine minimale Länge von 10 mm aufweist.

6. Sensor (10) gemäß einem der Ansprüche 1 bis 5, wobei
ein zweites Reservoir (22) mit dem Kanal (20) derart verbunden ist, dass es Anzeigeeinrichtungen (32) trennt, die verschiedene physikalische Einflüsse (F, T1, T2) anzeigen.

7. Sensor (10) gemäß Anspruch 6, wobei
der Kanal (20) zwei Flüssigkeits-/Luftgrenzflächen (32) enthält, wobei der Abstand (t2) zwischen den beiden Flüssigkeits-/Luftgrenzflächen (32) die Temperatur (T1, T2) des Sensors (10) wiedergibt.

8. Sensor (10) gemäß Anspruch 6 oder 7, wobei
der Kanal (20) zwei Flüssigkeits-/Luftgrenzflächen (32) enthält, wobei der Abstand (f) zwischen einer Flüssigkeits-/Luftgrenzfläche (32) und einem Reservoir (22) die Belastung (F) wiedergibt, die auf dieses Reservoir (22) ausgeübt wird.

9. Sensor (10) gemäß einem der Ansprüche 1 bis 7, wobei
das Reservoir (22) mindestens eine Vertiefung (18) aufweist, um die Volumenänderung zu vergrößern, wenn eine Belastung (F) auf das Reservoir (22) ausgeübt wird.

10. Sensor (10) gemäß einem der Ansprüche 1 bis 9, wobei
der Sensor (10) biokompatibel ist.

11. Sensor (10) gemäß Anspruch 10, wobei
der Sensor (10) biologisch abbaubar ist.

## Revendications

1. Détecteur (10) comprenant
- des premiers moyens (22, 42) qui réagissent à un impact physique (F, T1, T2) agissant sur eux ; et
- des moyens indicateurs (20 ; 44, 62) couplés aux premiers moyens (22, 42), les moyens indicateurs indiquant la magnitude de l'impact physique (F, T1, T2),
étant précisé que le détecteur (10) comprend un réservoir (22) et que les moyens indicateurs (20 ; 44, 62) comprennent un microcanal (20) pour recevoir un liquide (34) provenant du réservoir (22) et expulsé de celui-ci par l'impact physique (F, T1, T2),
**caractérisé en ce que** les moyens indicateurs (20 ; 44, 62) sont formés par une interface de contraste (32) qui forme un contraste entre deux milieux et qui est lisible par le son.

2. Détecteur (10) selon la revendication 1, étant précisé que l'un des deux milieux est un liquide (34), et l'autre un gaz.

3. Détecteur (10) selon l'une quelconque des revendications 1 ou 2, étant précisé que le microcanal (20) présente un tracé en spirale ou sinueux.

4. Détecteur (10) selon l'une quelconque des revendications précédentes, étant précisé que le microcanal (20) présente une largeur maximum de 1 mm, une hauteur maximum de 0,1 mm et une longueur maximum de 100 mm.

5. Détecteur (10) selon l'une quelconque des revendications précédentes, étant précisé que le microcanal (20) présente une largeur minimum de 0,01 mm, une hauteur minimum de 0,0001 mm et une longueur minimum de 10 mm.

6. Détecteur (10) selon l'une quelconque des revendications 1 à 5, étant précisé qu'un deuxième réservoir (22) est relié au canal (20) afin de séparer des moyens indicateurs (32) indiquant différents impacts physiques (F, T1, T2).

7. Détecteur (10) selon la revendication 6, étant précisé que le canal (20) contient deux interfaces liquide/air (32), la distance (t2) entre les deux interfaces liquide/air (32) représentant la température (T1, T2) du détecteur (10).

8. Détecteur (10) selon la revendication 6 ou 7, étant précisé que le canal (20) contient deux interfaces liquide/air (32), la distance (f) entre l'une des interfaces liquide/air (32) et un réservoir (22) représentant l'effort (F) appliqué à ce réservoir (22).

9. Détecteur (10) selon l'une quelconque des revendications 1 à 7, étant précisé que le réservoir (22) comporte au moins un creux (18) afin d'augmenter la variation de volume quand un effort (F) est appliqué sur le réservoir (22).

10. Détecteur (10) selon l'une quelconque des revendications 1 à 9, étant précisé que le détecteur (10) est biocompatible.

11. Détecteur (10) selon la revendication 10, étant précisé que le détecteur (10) est biodégradable.
